# EUROPÄISCHE PATENTSCHRIFT

(11) **EP 4 229 124 B1**
(45) Veröffentlichungstag und Bekanntmachung des Hinweises auf die Patenterteilung: **02.12.2026**
(21) Anmeldenummer: 21794128.5
(22) Anmeldetag: 14.10.2021
(51) Int. Cl.: C08J 11/16

(54) **VERFAHREN ZUR DEPOLYMERISATION VON POLYMERMASSEN UNTER ABBAU VON ORGANISCHEN HALOGEN-VERBINDUNGEN**
METHOD FOR DEPOLYMERIZING POLYMER MASSES WHILE DEGRADING ORGANIC HALOGEN COMPOUNDS
PROCÉDÉ DE DÉPOLYMÉRISATION DE MASSES POLYMÈRES TOUT EN DÉGRADANT DES COMPOSÉS HALOGÉNÉS ORGANIQUES

(30) Priorität: 19.10.2020 EP 20202633
(43) Veröffentlichungstag der Anmeldung: 23.08.2023
(73) Patentinhaber: INEOS Styrolution Group GmbH, 60325 Frankfurt (DE)
(72) Erfinder: NIESSNER, Norbert, 67159 Friedelsheim (DE); WILHELMUS, Bianca, 63456 Hanau (DE); KERSCHBAUMER, Hannes, 65812 Bad Soden am Taunus (DE); SCHMIDT, Hans-Werner, 95444 Bayreuth (DE); KOLB, Tristan, 95447 Bayreuth (DE); SCHEDL, Andreas, 95444 Bayreuth (DE)
(74) Vertreter: Jacobi, Markus Alexander
(86) Internationale Anmeldenummer: PCT/EP2021/078426
(87) Internationale Veröffentlichungsnummer: WO 2022/084138

(56) Entgegenhaltungen:
- WO-A1-2018/224482
- JP-A- 2000 119 440
- JUNG SU-HWA ET AL: "Fast pyrolysis of a waste fraction of high impact polystyrene (HIPS) containing brominated flame retardants in a fluidized bed reactor: The effects of various Ca-based additives (CaO, Ca(OH)2and oyster shells) on the removal of bromine", FUEL, vol. 95, 1 December 2012 (2012-12-01), pages 514 - 520, XP028889831, ISSN: 0016-2361, DOI: 10.1016/J.FUEL.2011.11.048
- HLAING Z.Z. ET AL: "Reduction of Bromine Compounds in the Pyrolysis Oil of Computer Casing Plastics Using Shell, Ca(OH)2 and NaOH", 3RD INTERNATIONAL CONFERENCE ON BIOTECHNOLOGY AND FOOD SCIENCE (ICBFS 2012) ELSEVIER SCIENCE BV, SARA BURGERHARTSTRAAT 25, PO BOX 211, 1000 AE AMSTERDAM, NETHERLANDS SERIES : APCBEE PROCEDIA (ISSN 2212-6708(PRINT)), vol. 10, 1 January 2014 (2014-01-01), Netherlands, pages 193 - 197, XP055865686, ISSN: 2212-6708, Retrieved from the Internet <URL:http://dx.doi.org/10.1016/j.apcbee.2014.10.037> DOI: 10.1016/j.apcbee.2014.10.037

## Beschreibung

### Geänderte Beschreibung

Die Erfindung betrifft ein Verfahren zur Verringerung des Gehalts an organischen Halogenverbindungen bei der Depolymerisation (Abbau) einer Polymermasse enthaltend organische Halogenverbindungen in Anwesenheit von basischen anorganischen Verbindungen. Ein Verfahren zur Rückgewinnung von Styrol-Monomeren aus einer Polymermasse (A) durch Pyrolyse einer Styrol-haltigen Masse unter Abbau der darin enthaltenen halogenhaltigen Verbindungen erweist sich als attraktiv zur Schonung von Ressourcen.

Der Übergang von einer Linear- zu einer Kreislaufwirtschaft ist angesichts von Klimawandel, Umweltverschmutzung, Bevölkerungswachstum und Ressourcenabhängigkeit sowohl ökologisch als auch ökonomisch erforderlich. In den 1990er Jahren wurden bereits intensive Anstrengungen zur Entwicklung von Verfahren für die rohstoffliche Wiederverwertung von Kunststoffabfällen unternommen, wobei es aufgrund ungelöster verfahrenstechnischer Problemstellungen und aus wirtschaftlichen Gründen bisher nicht zu großtechnischen Anwendungen kam. Aufgrund des allgemein wachsenden Umweltbewusstseins und einem steigenden Bedarf an nachhaltigen Lösungen wächst allerdings auch das Interesse an chemischem Recycling.

Nicht alle thermoplastischen Polymere sind gleichermaßen für das chemische Recycling geeignet. Bei der thermischen Zersetzung von Polyolefinen oder Polyestern entstehen Gemische aus u.a. Wachsen, Leichtöl und Gasen. Der Abbau von Polyethylenterephthalat (PET) führt zu organischen Säuren, hauptsächlich Benzoesäure und Terephthalsäure, die korrosiv sind, und auch eine Verstopfung des Reaktors verursachen können (G. Grause et al., Feedstock recycling of waste polymeric material, Journal of Material Cycles and Waste Management, 13(4), 2011, 265-282).

Im Fall von Polystyrol und anderen Styrol-haltigen Polymeren bzw. Polymermassen ist es möglich, diese Polymere in ihre Grundbestandteile, insbesondere Styrol-Monomere, zu depolymerisieren, weshalb Polystyrol und andere Styrol-Monomer-haltige Polymere ausgezeichnete Ausgangsmaterialien für das chemische Recycling bilden.

Ein technisches Problem ist, dass einige Styrol-haltige Polymermassen mit Flammschutzmitteln versehen sind, beispielsweise Schaumstoffe aus expandiertem Polystyrol (EPS), welche als Isoliermaterial im Bauwesen eingesetzt werden.

Auch schlagzähmodifiziertes Polystyrol (HIPS), welches in Elektrogerätegehäusen verwendet wird, oder andere Styrol(co)polymere werden ggf. mit halogenhaltigen Additiven versetzt.

Der Einsatz von Flammschutzmitteln soll die Entzündungsgefahr sowie die Flammenausbreitung im Falle eines Brandes reduzieren. Historisch wurde z.B. 1,2,5,6,9,10-Hexabromcyclododecan (HBCD) als Flammschutzmittel für EPS und HIPS verwendet. HBCD wurde im Mai 2013 in das Stockholmer Übereinkommen über persistente organische Schadstoffe aufgenommen. Für HBCD gilt ein weltweites Herstellungs- und Anwendungsverbot als Flammschutzmittel. In Altbauten und älteren Elektrogeräten ist HBCD jedoch noch weit verbreitet. Wenn diese Produkte einer Entsorgung zugeführt werden, ist es notwendig, das enthaltene HBCD unschädlich zu machen bzw. aus dem Resultat des Recyclings (Rezyklat) zu entfernen, falls das Polymer wieder für neue Produkte eingesetzt werden soll.

Bislang werden HBCD-haltige Produkte meistens thermisch verwertet, weil es keine etablierten Verfahren gibt, um HBCD soweit aus dem Rezyklat zu entfernen, dass die vom Gesetzgeber festgelegten Grenzwerte nicht überschritten werden.

Die japanische Veröffentlichung JP-B 3752101 bezieht sich auf ein Verfahren zur Trennung einer thermoplastischen Harzzusammensetzung in Flammschutzmittel und das thermoplastische Harz. Dieses Verfahren deckt somit ein Verfahren zur Behandlung einer Flammschutzmittel enthaltenden thermoplastischen Harzzusammensetzung ab, das folgende Schritte umfasst: Dispergieren einer thermoplastischen Styrolpolymer-Zusammensetzung, die ein Flammschutzmittel auf Brombasis enthält, in einem Lösemittel, um mindestens einen Teil des thermoplastischen Harzes zu lösen, sukzessives Entfernen von mindestens einem Teil des Flammschutzmittels des thermoplastischen Harzes aus der Lösung, in dem das Harz gelöst ist, und weiterhin Entfernen von mindestens einem Teil des thermoplastischen Harzes oder des Flammschutzmittels aus der Lösung, aus der das Flammschutzmittel oder das thermoplastische Harz entfernt wurde.

Das Patent US 6388050 bezieht sich auf ein Verfahren zur Behandlung einer Flammschutzmittel enthaltenden Harzzusammensetzung vom Styrol-Typ, das folgende Schritte umfasst: einen Lösungs- oder Dispergierschritt (a), bei dem eine ein Bromid-Flammschutzmittel enthaltende Harzzusammensetzung vom Styrol-Typ mit einem einzelnen Lösemittel in Kontakt gebracht wird, um mindestens einen Teil des Flammschutzmittels in dem Lösemittel zu lösen oder zu dispergieren, einen Trennungsschritt (b), bei dem eine Lösung oder Dispersion des Flammschutzmittels nach Schritt (a) abgetrennt wird, einen Trocknungsschritt (c), bei dem die Harzzusammensetzung vom Styroltyp, aus der das Flammschutzmittel nach Schritt (b) abgetrennt wird, getrocknet wird.

US 7435772 beschreibt die Behandlung einer Harzzusammensetzung, enthaltend ein bromiertes Flammschutzmittel und Antimon enthaltendes Flammschutzmittel, mit zwei Lösemitteln bei einer Temperatur zwischen der Glasübergangstemperatur des Polymers und des Siedepunkts des jeweiligen Lösemittels, in zwei aufeinander folgenden Schritten.

CN-A 105237799 bezieht sich auf ein Verfahren zur Abtrennung von polybromierten Diphenylethern aus Polymerzusammensetzungen mittels eines Lösemittels.

US 8138232 beschreibt ein Verfahren zum Recycling einer Zusammensetzung aus mindestens zwei Polymeren oder Copolymeren auf Styrolbasis, bei dem die Zusammensetzung mit einem Lösemittel versetzt und anschließend ein Fällmittel zugesetzt wird.

JP-A 2016010906 bezieht sich auf ein Verfahren zur Entfernung von HBCD aus einer geschäumten Polystyrolzusammensetzung mit zwei Lösemitteln, eines, das HBCD löst, aber nicht Polystyrol, und ein zweites, das Polystyrol löst.

Die vorgenannten Veröffentlichungen beschreiben alle eine Abreicherung des halogenierten Flammschutzmittels durch Anwendung eines Lösemittels. Dabei wird das bromierte Flammschutzmittel jedoch nicht unschädlich gemacht oder vollständig entfernt.

JP-A 2000290424 bezieht sich auf ein Verfahren zur Wiederverwertung einer thermoplastischen Harzzusammensetzung, die ein Flammschutzmittel auf Brombasis enthält, wobei die Harzzusammensetzung mit Wasser oder einem Alkohol zur Beschleunigung der Debromierungsreaktion des Flammschutzmittels zur Entfernung des Broms in Kontakt gebracht wird. Dieses Verfahren zur Wiederverwertung der thermoplastischen Harzzusammensetzung, die das Flammschutzmittel auf Brombasis enthält, umfasst zudem das In-Kontakt-bringen der Harzzusammensetzung mit einem Metallhydroxid, einem Metallcarbonat oder Octylalkohol, um die entsprechenden Bromidsalze oder Octylbromid zu erzeugen, und die anschließende Abspaltung der erzeugten Bromide oder des erzeugten Octylbromids von dem Harz.

US 6903242 bezieht sich auf ein Verfahren zur Dehalogenierungsbehandlung einer halogenhaltigen Flammschutzmittel-Harz-Zusammensetzung, das einen Schritt umfasst, bei dem die halogenhaltige Flammschutzmittel-Harz-Zusammensetzung mit einer Materialmischung, die ein Dehalogenierungs-Material und ein die Dehalogenierung förderndes Material enthält, bei einer Temperatur, die unter der Wärmezersetzungstemperatur der Harzzusammensetzung liegt, durch Kneten der Mischung und gleichzeitige Scherkraftanwendung in einem biaxialen Knetextruder, einem Kneter oder Drehwalzen in Kontakt gebracht wird.

DE-A 10 2016 125 506 bezieht sich auf ein Verfahren zur Wiederverwertung von EPS-Schaumstoffen, die halogenhaltige Flammschutzmittel umfassen, wobei die EPS-Schaumstoffe in Gegenwart anderer Ausgangsmaterialien extrudiert, abgekühlt und weiter zu Partikeln zerkleinert werden. Der Extrusionsschritt erfolgt in Gegenwart mindestens eines Halogenfängers.

EP-A 2839863 bezieht sich auf ein Verfahren zur Deaktivierung bromierter Verbindungen mittels UV-Strahlung, ohne Verwendung eines Lösemittels.

Den vorgenannten Veröffentlichungen ist gemein, dass die verwendeten Halogenfänger meist nur für eine Inaktivierung von bromierten Flammschutzmitteln sorgen. Die neu entstehenden Bromverbindungen, beispielsweise CaBr₂, verbleiben im Produkt und können so die Produkteigenschaften beeinflussen, oder sie müssen aufwendig aus dem Produkt entfernt werden.

Calciumverbindungen sind bekannt dafür, dass sie mit Halogenverbindungen aus Polymerzusammensetzungen reagieren, z.B. können CaCO₃ und CaO mit dem bei der Pyrolyse von Polyvinylchlorid (PVC) entstehendem Chlor reagieren (K. Ragaert et al., Mechanical and chemical recycling of solid plastic waste, in: Waste Management, 69, 2017, 24-58). CaO und Ca(OH)₂ können ebenfalls mit den Bromverbindungen reagieren, die bei der Pyrolyse einer Zusammensetzung aus Polystyrol und bromierten Flammschutzmitteln entstehen (S. H. Jung et al., Fast pyrolysis of a waste fraction of high impact polystyrene (HIPS) containing brominated flame retardants in a fluidized bed reactor: The effects of various Ca-based additives (CaO, Ca(OH)2 and oyster shells) on the removal of bromine, in: Fuel, 95, 2012, 514-520). Weitere Verfahren zur Pyrolyse von halogenhaltigen Polymermassen in Anwesenheit von Oxiden, Hydroxiden oder Carbonaten sind in JP 2000 119440 und in "Reduction of Bromine Compounds in the Pyrolysis Oil of Computer Casing Plastics Using Shell, Ca(OH)₂ and NaOH" (Z. Z. Hlaing et al., APCBEE Procedia, 10, 2014, 193-197) beschrieben.

Alkali- und Erdalkalioxide wie MgO, CaO, BaO und K₂O sind neben Zeolithen SiO₂/Al₂O₃ und weiteren festen Säuren und Basen bekannt dafür, dass sie die Zersetzung von Polystyrol bei der Pyrolyse fördern (Z. Zhang et al., Chemical Recycling of Waste Polystyrene into Styrene over Solid Acids and Bases, in: Industrial & Engineering Chemistry Research, 34, 1995, 4514-4519).

JP 2545748 beschreibt die Verwendung von Metalloxiden als Katalysator zur Zersetzung von Polystyrol. Es wird jedoch kein flammgeschütztes Polystyrol beschrieben.

Zeolithe und Eisen-beladene Zeolithe sind besonders effektive Bromfänger bei der Pyrolyse von Zusammensetzungen aus Polystyrol und bromierten Flammschutzmitteln (H. Wu et al., Fuel Oil Production from Two-Stage Pyrolysis-Catalytic Reforming of Brominated High Impact Polystyrene Using Zeolite and Iron Oxide Loaded Zeolite Catalysts, Open Journal of Ecology, 05(04), 2015, 136-146).

Wenn Polystyrol ausreichend thermisch behandelt wird, zersetzt es sich zu Styrol-Monomeren. Eine unvollständige Zersetzung führt auch zur Bildung von z.B. Styrol-Dimeren, -Trimeren und anderen Oligomeren. Wenn die Zersetzungsbedingungen zu harsch sind, können Nebenprodukte, z.B. Benzol, Toluol, Ethylbenzol, Cumol und alpha-Methylstyrol gebildet werden. Die Mengen dieser Reaktionsprodukte können variieren. Sie hängen insbesondere von den Reaktionsbedingungen und den verwendeten Rohstoffen ab (s. C. Bouster et al., Study of the pyrolysis of polystyrenes: Kinetics of thermal decomposition, in: Journal of Analytical and Applied Pyrolysis, 1 (1980) 297-313 und C. Bouster et al., Evolution of the product yield with temperature and molecular weight in the pyrolysis of polystyrene, Journal of Analytical and Applied Pyrolysis 15 (1989) 249-259).

Die gewonnenen Styrol-Monomere können ggf. für ein neues Polymerisationsverfahren verwendet werden. Styrol-Oligomere können jedoch den Polymerisationsprozess stören, da sie bereits in geringen Mengen wichtige Eigenschaften des Polymers beeinflussen. Dies gilt auch für andere Nebenprodukte. Daher müssen die Styrol-Monomere von anderen Komponenten des Produktgemisches getrennt werden, um eine hohe Produktqualität zu gewährleisten.

EP-A 3635043 (INEOS Styrolution) beschreibt ein Verfahren zur Wiedergewinnung von Styrol aus Styrol-haltigen Kunststoffabfällen mittels Depolymerisation. Auf die Abreicherung von halogenierten Fremdstoffen wird jedoch nicht abgezielt.

Es besteht derzeit ein hoher Bedarf an einem auch im großen Maßstab durchführbaren Verfahren zur Herstellung von Styrol-Monomeren aus flammgeschützten Styrol-haltigen Polymermassen in einer Reinheit, die den gesetzlichen Vorgaben bezüglich des Gehalts an halogenhaltigen Flammschutzmitteln genügen.

Eine Aufgabe der vorliegenden Erfindung ist daher die Bereitstellung eines Verfahrens zur Entfernung oder zum Unschädlichmachen von Halogenverbindungen bei der Pyrolyse eines Styrol-haltigen Polymers bzw. einer Polymermasse in Anwesenheit eines halogenierten Flammschutzmittels.

Eine weitere Aufgabe der vorliegenden Erfindung ist die Bereitstellung eines Verfahrens zur Wiederverwertung von Styrol-haltigen Kunststoffen, die halogenhaltige Flammschutzmittel umfassen, bei dem das entstehende Produkt die gesetzlichen Vorgaben bezüglich des Gehalts an halogenhaltigen Flammschutzmitteln erfüllt und bei dem die physikalischen Eigenschaften des entstehenden Produkts nicht negativ durch die im Produkt verbleibenden Nebenprodukte beeinflusst werden.

Die vorliegende Erfindung bezieht sich auch auf ein Verfahren zur Wiederverwertung von Styrol-haltigen Kunststoffen, die halogenhaltige Flammschutzmittel umfassen, wobei die Styrol-haltigen Polymere in einem Reaktor depolymerisiert werden, und der Depolymerisationsschritt in Gegenwart mindestens eines Halogenfängers erfolgt.

Die Erfinder der vorliegenden Erfindung haben festgestellt, dass sich mit Hilfe eines solchen Verfahrens die erwähnten Aufgaben in technisch praktikabler Weise bewältigen lassen.

Überraschend wurde gefunden, dass basische anorganische Oxide wie BaO, CaO und MgO sowohl die Depolymerisation von Styrol-haltigen Polymerzusammensetzungen begünstigen als auch den Abbau von bromierten Flammschutzmitteln fördern. Durch ein Abtrennen der gasförmigen Reaktionsprodukte von der Reaktionsmischung kann eine Verminderung der Menge an bromierten Verbindungen im Produkt erzielt werden.

Ein Gegenstand der Erfindung ist daher ein Verfahren zur Rückgewinnung von Styrol-Monomeren aus einer Polymermasse (A) durch Pyrolyse unter Abbau der darin enthaltenen halogenhaltigen Verbindungen, umfassend die Schritte:
a) Einbringen von:
   I) 25 bis 99,95 Gew.-%, bezogen auf das Gesamtgewicht von (A) und (B), einer Polymermasse (A) umfassend
      A1) 30 bis 99,9999 Gew.-%, bezogen auf das Gesamtgewicht der Polymermasse (A), mindestens eines Styrol-haltigen Polymers (A1) umfassend
         la) 10 bis 100 Gew.-%, bezogen auf das Gesamtgewicht des Styrol-haltigen Polymers (A1), an Wiederholungseinheiten (la), die von Styrol stammen; und
         lb) 0 bis 90 Gew.-%, bezogen auf das Gesamtgewicht des Styrol-haltigen Polymers (A1), an Kautschuk und/oder an Wiederholungseinheiten (Ib), die sich von den Wiederholungseinheiten (la) unterscheiden und den Pyrolyseprozess nicht unterbrechen, bevorzugt ausgewählt aus Wiederholungseinheiten (Ib), die von Acrylnitril, Acrylestern, Methylmethacrylat oder alpha-Methylstyrol stammen, oder Kombinationen davon;
      A2) 0 bis 69,9999 Gew.-%, bezogen auf das Gesamtgewicht der Polymermasse (A), weiterer Polymere (A2), die sich von dem mindestens einen Styrol-haltigen Polymer (A1) unterscheiden und den Pyrolyseprozess nicht unterbrechen, bevorzugt ausgewählt aus der Gruppe bestehend aus Polyolefinen, Polycarbonaten, Polyestern, Polyamiden, Polyalkyl(meth)acrylaten, Polyurethanen und Kombinationen davon, besonders bevorzugt ausgewählt aus der Gruppe bestehend aus Polyethylen, Polypropylen, Polymethylmethacrylat und Kombinationen davon, besonders bevorzugt Polymethylmethacrylat;
      A3) 0,0001 bis 50 Gew.-%, bezogen auf das Gesamtgewicht der Polymermasse (A), mindestens einer halogenhaltigen Verbindung (A3); und
      A4) 0 bis 50 Gew.-%, bezogen auf das Gesamtgewicht der Polymermasse (A), weiterer Komponenten (A4), die den Pyrolyseprozess nicht unterbrechen, bevorzugt ausgewählt aus der Gruppe bestehend aus Füllstoffen, Stabilisatoren, anderen organischen und/oder anorganischen Additiven, Feuchtigkeit und anderen organischen und/oder anorganischen Fremdstoffen; und
   II)0,05 bis 75 Gew.-%, bezogen auf das Gesamtgewicht von (A) und (B), mindestens einer anorganischen basischen Verbindung (B),
      in die Reaktionszone (R) eines Pyrolysereaktors (P);
b) thermisches Spalten des in der Polymermasse (A) enthaltenen Styrol-haltigen Polymers (A1) in der Reaktionszone (R) des Pyrolysereaktors (P), um ein Produktgemisch (G), enthaltend Styrol-Monomere und weitere Komponenten, zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (G'), enthaltend Styrol-Monomere und weitere Komponenten, zu erhalten; und
e) Abtrennen einer Styrol-Monomer-haltigen Fraktion von den weiteren Komponenten des in Schritt d) erhaltenen kondensierten Produktgemisches (G'), um eine Styrol-Monomer-haltige Flüssigkeit zu erhalten,
dadurch gekennzeichnet, dass in der Polymermasse (A) und in der anorganischen basischen Verbindung (B), kein Antimonoxid (Sb₂O₃) vorhanden ist.

Die thermische Zersetzung (Depolymerisation) der Polymermasse (A) kann im Prinzip in jedem geeigneten Reaktor als Pyrolysereaktor (P) stattfinden, in dem die zur Zersetzung erforderliche Temperatur erreicht werden kann. Beispielsweise kann der Pyrolysereaktor (P) ausgewählt sein aus der Gruppe bestehend aus Extrudern, Batchreaktoren, Drehrohren, Mikrowellenreaktoren, Rohrbündelreaktoren, Vortex-Reaktoren und Wirbelschichtreaktoren.

Beispielsweise kann die thermische Zersetzung in einem Drehrohrofen durchgeführt werden. Drehrohröfen sind z.B. in EP-A 1481957 beschrieben. Die thermische Zersetzung kann auch in Extrudern stattfinden; diese sind z.B. in EP-A 1966291 beschrieben. Solche Reaktoren können mit oder ohne Gasstrom betrieben werden, etwa Trägergas oder Gas als Reaktionsmedium.

Der Pyrolysereaktor (P) kann demnach eine beliebige bekannte Art von Pyrolysereaktor sein, unter der Voraussetzung, dass der Aufbau des Pyrolysereaktors eine Einstellung der Temperatur in der Reaktionszone (R) des Pyrolysereaktors (P) ermöglicht.

Bevorzugt ist der Pyrolysereaktor (P) ausgewählt aus der Gruppe bestehend aus Doppelschneckenextrudern, kontinuierlichen Rührtankreaktoren, Vortex-Reaktoren und Wirbelschichtreaktoren. Wenn der Pyrolysereaktor ein Wirbelschichtreaktor ist, kann es vorteilhaft sein, wenn die Wirbelschicht ganz oder teilweise aus der mindestens einen anorganischen basischen Verbindung (B) besteht.

Die Einstellung der Temperatur in der Reaktionszone (R) des Pyrolysereaktors (P) kann auf beliebige Art und Weise erfolgen. Beispielsweise kann die Temperatureinstellung durch Mikrowelleneinstrahlung, mit Hilfe von Wärmetauschern, Gasbrennern, resistiven Heizleitern (Widerstandsheizung), oder durch Einleiten von überhitztem Gas, insbesondere Wasserdampf, jeweils allein oder in Kombination erfolgen. In einer Ausführungsform erfolgt die Einstellung der Temperatur mit Hilfe von resistiven Heizleitern, die mit der Wand der Reaktionszone (R) des Pyrolysereaktors (P) in Kontakt stehen.

Alternativ kann die Einstellung der Temperatur mit Hilfe von Wasserdampf erfolgen, der durch Verdampfen von Wasser zur Verfügung gestellt und durch einen Dampfüberhitzer auf die gewünschte Temperatur gebracht wird.

Vorzugsweise wird die Temperatur in der Reaktionszone (R) des Pyrolyserealtors (P) dadurch eingestellt, dass die Reaktionszone (R) durch eine Mikrowellen-unterstützte Heizung beheizt wird.

Die Temperatur in der Reaktionszone (R) des Pyrolysereaktors (P) wird bevorzugt auf eine Temperatur von 250 °C bis 1000 °C, besonders bevorzugt von 300 °C bis 700 °C, ganz besonders bevorzugt von 380 °C bis 650 °C, insbesondere bevorzugt von 400 °C bis 600 °C eingestellt.

Das Einbringen der Polymermasse (A) und der mindestens einen anorganischen basischen Verbindung (B) in die Reaktionszone (R) des Pyrolysereaktors (P) in Schritt a) kann auf beliebige Art und Weise erfolgen. Beispielsweise kann das Einbringen durch manuelle oder maschinelle Zugabe der Komponenten in die Reaktionszone (R) erfolgen oder durch pneumatische Zufuhr der Komponenten in die Reaktionszone (R).

In einer weiteren Ausführungsform werden die Komponenten vor dem Einbringen in die Reaktionszone (R) miteinander compoundiert. Dies ist insbesondere vorteilhaft, wenn die Polymermasse (A) vor dem Einbringen in die Reaktionszone (R) einer Schmelzefiltration unterzogen werden soll.

In einer weiteren Ausführungsform wird die anorganische basische Verbindung (B) nur mit einem Teil der Polymermasse (A) compoundiert und als Masterbatch mit einer weiteren Menge Polymermasse (A) in die Reaktionszone (R) eingebracht.

Das Einbringen der Komponenten kann kontinuierlich oder diskontinuierlich, d. h. durch stetige Zufuhr oder in einer oder mehreren Portionen erfolgen. Bevorzugt erfolgt das Einbringen der Polymerasse (A) und der mindestens einen anorganischen basischen Verbindung (B) in die Reaktionszone (R) des Pyrolysereaktors (P) kontinuierlich, besonders bevorzugt in einer Geschwindigkeit, die den Verbrauch der jeweiligen Komponente bei der Pyrolyse kompensiert.

Die Polymermasse (A) wird in einer Menge von 25 bis 99,95 Gew.-%, bevorzugt in einer Menge von 50 bis 99,9 Gew.-%, besonders bevorzugt in einer Menge von 80 bis 99,5 Gew.-%, ganz besonders bevorzugt in einer Menge von 90 bis 99 Gew.-%, insbesondere bevorzugt in einer Menge von 92 bis 98 Gew.-%, bezogen auf das Gesamtgewicht von (A) und (B), in die Reaktionszone (R) des Pyrolysereaktors (P) eingebracht. Entsprechend wird die mindestens eine anorganische basische Verbindung (B) in einer Menge von 0,05 bis 75 Gew.-%, bevorzugt in einer Menge von 0,1 bis 50 Gew.-%, besonders bevorzugt in einer Menge von 0,5 bis 20 Gew.-%, ganz besonders bevorzugt in einer Menge von 1 bis 10 Gew.-%, insbesondere bevorzugt in einer Menge von 2 bis 8 Gew.-%, bezogen auf das Gesamtgewicht von (A) und (B), in die Reaktionszone des Pyrolysereaktors (P) eingebracht.

In einer Ausführungsform können zusätzlich zu der Polymermasse (A) und der mindestens einen anorganischen basischen Verbindung (B) auch weitere Komponenten, die das Verfahren nicht unterbrechen und/oder stören, insbesondere Verfahrenshilfsmittel in dem Verfahren eingesetzt werden. In einer weiteren Ausführungsform werden in dem Verfahren außer der Polymermasse (A) und der mindestens einen anorganischen basischen Verbindung (B) keine weiteren Komponenten eingesetzt.

Die bei dem erfindungsgemäßen Verfahren eingesetzte Polymermasse (A) enthält oft A1) 30 bis 99,9999 Gew.-%, bevorzugt 50 bis 99,999 Gew.-%, besonders bevorzugt 70 bis 99,99 Gew.-%, ganz besonders bevorzugt 90 bis 99,9 Gew.-%, bezogen auf das Gesamtgewicht der Polymermasse (A), mindestens eines Styrol-haltigen Polymers (A1); A2) 0 bis 69,9999 Gew.-%, bevorzugt 0 bis 49,999 Gew.-%, besonders bevorzugt 0 bis 29,99 Gew.-%, ganz besonders bevorzugt 0 bis 9,9 Gew.-%, bezogen auf das Gesamtgewicht der Polymermasse (A), weiterer Polymere (A2), die sich von dem mindestens einen Styrol-haltigen Polymer (A1) unterscheiden und den Pyrolyseprozess nicht unterbrechen; A3) 0,0001 bis 50 Gew.-%, bevorzugt 0,001 bis 20 Gew.-%, besonders bevorzugt 0,01 bis 10 Gew.-%, ganz besonders bevorzugt 0,1 bis 5 Gew.-%, bezogen auf das Gesamtgewicht der Polymermasse (A), mindestens einer halogenhaltigen Verbindung (A3); und A4) 0 bis 50 Gew.-%, bevorzugt 0 bis 30 Gew.-%, besonders bevorzugt 0 bis 10 Gew.-%, ganz besonders bevorzugt 0 bis 5 Gew.-%, bezogen auf das Gesamtgewicht der Polymermasse (A), weiterer Komponenten (A4), die den Pyrolyseprozess nicht unterbrechen.

Entsprechend beträgt die Menge an mindestens einer halogenhaltigen Verbindung (A3) in der Polymermasse (A) mindestens 1 ppm, bevorzugt mindestens 10 ppm, besonders bevorzugt mindestens 100 ppm, ganz besonders bevorzugt mindestens 1000 ppm.

In einer Ausführungsform besteht die Polymermasse (A) aus dem mindestens einen Styrol-haltigen Polymer (A1), der mindestens einen halogenhaltigen Verbindung (A3), optional weiteren Polymeren (A2) und optional weiteren Komponenten (A4). In einer weiteren Ausführungsform besteht die Polymermasse (A) nur aus dem mindestens einen Styrol-haltigen Polymer (A1) und der mindestens einen halogenhaltigen Verbindung (A3).

Bei dem mindestens einen Styrol-haltigen Polymer (A1) handelt es sich um ein Polymer umfassend
la) 10 bis 100 Gew.-%, bevorzugt 30 bis 100 Gew.-%, besonders bevorzugt 50 bis 100 Gew.-%, ganz besonders bevorzugt 70 bis 100 Gew.-%, bezogen auf das Gesamtgewicht des Styrol-haltigen Polymers (A1), an Wiederholungseinheiten (la), die von Styrol stammen; und
Ib) 0 bis 90 Gew.-%, bevorzugt 0 bis 70 Gew.-%, besonders bevorzugt 0 bis 50 Gew.-%, ganz besonders bevorzugt 0 bis 30 Gew.-%, bezogen auf das Gesamtgewicht des Styrol-haltigen Polymers (A1), an Kautschuk und/oder an Wiederholungseinheiten (Ib), die sich von den Wiederholungseinheiten (la) unterscheiden und den Pyrolyseprozess nicht unterbrechen.

Der Kautschuk, sofern in dem Styrol-haltigen Polymer (A1) vorhanden, kann beispielsweise ausgewählt sein aus der Gruppe bestehend aus Dien-Kautschuk, Acrylat-Kautschuk, Ethylen-Propylen-Dien-Kautschuk (EPDM-Kautschuk), Silikonkautschuk, Naturkautschuk, Verbundkautschuken daraus und Kombinationen der vorgenannten Kautschuke. Bevorzugt ist der Kautschuk, sofern vorhanden, ausgewählt aus Butadien-Kautschuk, Isopren-Kautschuk, Butylacrylat-Kautschuk, Verbundkautschuken daraus und Kombinationen dieser Kautschuke. Besonders bevorzugt ist der Kautschuk, sofern vorhanden, ausgewählt aus Butadien-Kautschuk und n-Butylacrylat-Kautschuk. Wenn Kautschuk in dem Styrol-haltigen Polymer (A1) enthalten ist, beträgt dessen Menge häufig mindestens 0,01 Gew.-%, bezogen auf das Gesamtgewicht des Styrol-haltigen Polymers (A1).

Die Wiederholungseinheiten (Ib), sofern in dem Styrol-haltigen Polymer (A1) vorhanden, stammen von Comonomeren wie beispielsweise Acrylnitril, Vinylchlorid, Alkylacrylaten, Alkylmethacrylaten, alpha-Methylstyrol, Maleinsäureanhydrid, Phenyl-Maleinimid, Butadien, Ethylen, alpha-Olefinen oder Kombinationen davon. Bevorzugt stammen die Wiederholungseinheiten (Ib), sofern in dem Styrol-haltigen Polymer (A1) vorhanden, von Comonomeren ausgewählt aus der Gruppe bestehend aus Acrylnitril, Acrylestern, Methylmethacrylat, alpha-Methylstyrol und Kombinationen davon. Wenn Wiederholungseinheiten (Ib) in dem Styrol-haltigen Polymer (A1) enthalten sind, beträgt deren Menge mindestens 1 Gew.-%, bevorzugt mindestens 5 Gew.-%, besonders bevorzugt mindestens 10 Gew.-%, ganz besonders bevorzugt mindestens 20 Gew.-%, bezogen auf das Gesamtgewicht des Styrol-haltigen Polymers (A1). Vorzugsweise enthält das Styrol-haltige Polymer (A1) keine Wiederholungseinheiten (Ib). Wenn Wiederholungseinheiten (Ib) in dem Styrol-haltigen Polymer (A1) enthalten sind, beträgt deren Menge häufig mindestens 0,01 Gew.-%, bezogen auf das Gesamtgewicht des Styrol-haltigen Polymers (A1).

Bevorzugt ist das Styrol-haltige Polymer (A1) ausgewählt aus der Gruppe bestehend aus Styrol-Homopolymeren (Polystyrol), Styrol-Copolymeren wie Styrol-Acrylnitril-Copolymeren, Styrol-alpha-Methylstyrol-Copolymeren, Styrol-Methylmethacrylat-Copolymeren, Styrol-alpha-Methylstyrol-Methylmethacrylat-Copolymeren, Styrol-alpha-Methylstyrol-Acrylnitril-Copolymeren, Styrol-Acrylnitril-Maleinsäureanhydrid-Copolymeren, Styrol-Acrylnitril-Phenylmaleinimid-Copolymeren, und deren Pfropfcopolymeren mit kautschukartigen Polymeren wie Acrylnitril-Butadien-Styrol-Pfropfcopolymeren (ABS), Acrylnitril-Styrol-Alkyl(meth)acrylat-Pfropfcopolymeren (ASA), Styrol-alpha-Methylstyrol-Acrylnitril-Methylmethacrylat-Copolymeren, Styrol-alpha-Methylstyrol-Acrylnitril-t-Butylmethacrylat-Copolymeren und Styrol-Acrylnitril-t-Butylmethacrylat-Copolymeren.

Die weiteren Polymere (A2), sofern in der Polymermasse (A) vorhanden, unterscheiden sich von dem mindestens einen Styrol-haltigen Polymer (A1) und unterbrechen den Pyrolyseprozess nicht. Sie sind beispielsweise ausgewählt aus der Gruppe bestehend aus Duroplasten, Harzen, Polyolefinen, Polycarbonaten, Polyestern, Polyamiden, Polyalkyl(meth)acrylaten, Polyurethanen, Poly-alpha-Methylstyrol, alpha-Methylstyrol-Methylmethacrylat-Copolymeren, alpha-Methylstyrol-Acrylnitril-Copolymeren (AMSAN), und deren Pfropfcopolymeren mit kautschukartigen Polymeren wie alpha-Methylstyrol-Acrylnitril-Methylmethacrylat-Copolymeren, alpha-Methylstyrol-Acrylnitril-t-Butylmethacrylat-Copolymeren, Acrylnitril-Butadien-alpha-Methylstyrol-Pfropfcopolymeren und Kombinationen davon. Bevorzugt sind die weiteren Polymere (A2), sofern in der Polymermasse (A) vorhanden, ausgewäht aus der Gruppe bestehend aus Polyolefinen, Polycarbonaten, Polyestern, Polyamiden, Polyalkyl(meth)acrylaten, Polyurethanen und Kombinationen davon, besonders bevorzugt ausgewählt aus der Gruppe bestehend aus Polyethylen, Polypropylen, Polymethylmethacrylat und Kombinationen davon, ganz besonders bevorzugt Polymethylmethacrylat. Wenn weitere Polymere (A2) in der Polymermasse (A) enthalten sind, beträgt deren Menge häufig mindestens 0,01 Gew.-%, bezogen auf das Gesamtgewicht der Polymermasse (A).

Als Polyolefine, wenn vorhanden, kommen beliebige Polyolefine in Frage, beispielsweise Polyethylen- oder Polypropylen-Derivate wie PE-LD (Low Density Polyethylen), PE-LLD (Linear Low Density Polyethylen), PE-HD (High Density Polyethylen), Metallocen-Polyethylene, Ethylen-Copolymere wie Poly(ethylen-co-vinylacetat), Ethylen-Buten-, Ethylen-Hexen-, Ethylen-Octen-Copolymere, sowie Cycloolefincopolymere, Homo- oder Copolymere des Propylens, Metallocen-katalysierte Polypropylene sowie Copolymere des Propylens mit anderen, dem Fachmann bekannten Comonomeren, und Mischungen davon. Bevorzugte Polyolefine sind Homopolymere von Ethylen, Homopolymeren von Propylen, Copolymeren von Ethylen und Propylen und Mischungen davon.

Als Polyester, wenn vorhanden, kommen beliebige Polyester in Frage, beispielsweise Polykondensationsprodukte von Dicarbonsäuren enthaltend 4 bis 16 Kohlenstoffatome mit Diolen enthaltend 2 bis 8 Kohlenstoffatome oder Polykondensationsprodukte von Hydroxycarbonsäuren enthaltend 2 bis 6 Kohlenstoffatome. Hierzu gehören unter anderem Polyalkylenadipate, wie Polyethylenadipat und Polybutylenadipat, Polyalkylenterephthalate, wie Polyethylenterephthalat und Polybutylenterephthalat, Polymilchsäure, Polyhydroxybutyrate, Polycaprolactone und Polyvalerolactone. Bevorzugte Polyester, wenn vorhanden, sind Polyethylenterephthalat (PET) und Polybutylenterephthalat (PBT), insbesondere Polyethylenterephthalat.

Als Duroplaste oder Harze, wenn vorhanden, kommen beliebige Duroplaste oder Harze infrage, beispielsweise Phenolharze, Harnstoffharze, Melaminharze, Alkydharze oder Epoxidharze und/oder deren jeweiligen Härtungsprodukte.

In einer Ausführungsform ist das Styrol-haltige Polymer (A1) Polystyrol und gegebenenfalls das weitere Polymer (A2) Polymethylmethacrylat.

Bei der halogenhaltigen Verbindung (A3), die vorzugsweise eine halogenhaltige organische Verbindung (A3) ist, handelt es sich beispielsweise um ein halogenhaltiges Flammschutzmittel.

Beispiele für das halogenhaltige Flammschutzmittel sind bromierte Flammschutzmittel und chlorierte Flammschutzmittel. Bevorzugte bromierte Flammschutzmittel sind ausgewählt aus der Gruppe bestehend aus polybromierten Diphenylethern (Penta-, Octa-, Deca-Bromdiphenylether), Decabromdiphenylethan (DBDPE), Tetrabrombisphenol A (TBBPA), polybromierten Biphenylen (PBB), 2,4,6-Tribromphenol (TBP), 1,2-Bis(2,4,6-tribromphenoxy)ethan (BTBPE) und 1,2,5,6,9,10-Hexabromcyclododecan (HBCD) sowie bromierten Styrol-Butadien-Copolymeren (FR-122P) und bromiertem Polystyrol. Bevorzugte chlorierte Flammschutzmittel sind ausgewählt aus der Gruppe bestehend aus Chlorparaffinen und Mirex (1,1a,2,2,3,3a,4,5,5a,5b,6-dodecachloroacta-hydro-1,3,4-metheno-1H-cyclobuta[cd]pentalen). Besonders bevorzugt ist die halogenierte Verbindung (A3) ein bromiertes Flammschutzmittel, vorzugsweise Hexabromcyclododecan (HBCD) und/oder polybromiertes Styrol-Butadien-Copolymer.

Bei den weiteren Komponenten (A4), sofern in der Polymermasse (A) vorhanden, kann es sich um beliebige Stoffe handeln, die den Pyrolyseprozess nicht unterbrechen und sich von der halogenhaltigen Verbindung (A3) und den Polymeren (A1) und (A2) unterscheiden. Häufig sind die weiteren Komponenten (A4) übliche Kunststoffadditive und - hilfsstoffe.

Bevorzugt sind die weiteren Komponenten (A4) ausgewählt aus der Gruppe bestehend aus Füllstoffen, Stabilisatoren, anderen organischen und/oder anorganischen Additiven, Feuchtigkeit und anderen organischen und/oder anorganischen Fremdstoffen. Wenn weitere Komponenten (A4) in der Polymermasse (A) enthalten sind, beträgt deren Menge häufig mindestens 0,001 Gew.-%, bezogen auf das Gesamtgewicht der Polymermasse (A).

Die weiteren Komponenten (A4) können beispielsweise ausgewählt sein aus der Gruppe bestehend aus Antioxidantien, UV-Stabilisatoren, Peroxidzerstörern, Antistatika, Gleitmitteln, Entformungsmitteln, nicht-halogenhaltigen Flammschutzmitteln, Füll- oder Verstärkerstoffen (Glasfasern, Kohlefasern, Graphit etc.), Farbmitteln, Keimbildungsmitteln, Antiblockmitteln, Verarbeitungshilfsmitteln, Weichmachern, nicht-halogenierten Flammschutzmitteln und Kombinationen aus zwei oder mehr daraus.

Als Beispiele für Oxidationsverzögerer und Wärmestabilisatoren seien Halogenide von Metallen der Gruppe I des periodischen Systems, z.B. Natrium-, Kalium- und/oder Lithiumhalogenide, ggf. in Verbindung mit Kupfer-(I)-Halogeniden, z.B. Chloriden, Bromiden, Jodiden, sterisch gehinderte Phenole, Hydrochinone, verschiedene substituierte Vertreter dieser Gruppen und deren Mischungen in Konzentrationen bis zu 2 Gew.-%, bezogen auf das Gesamtgewicht der Polymermasse (A) genannt.

Als UV-Stabilisatoren, die im Allgemeinen in Mengen bis zu 2 Gew.-%, bezogen auf das Gesamtgewicht der Polymermasse (A) enthalten sind, seien verschiedene substituierte Resorcine, Salicylate, Benzotriazole und Benzophenone genannt.

Weiterhin können organische Farbstoffe wie Nigrosin, Pigmente wie Titandioxid, Phthalocyanine, Ultramarinblau und Ruß als Farbstoffe in der Polymermasse (A) enthalten sein, sowie faser- und pulverförmige Füllstoffe und Verstärkungsmittel. Beispiele für letztere sind Kohlenstofffasern, Glasfasern, amorphe Kieselsäure, Calciumsilicat (Wollastonit), Aluminiumsilicat, Magnesiumcarbonat, Kaolin, Kreide, gepulverter Quarz, Glimmer und Feldspat.

Als Keimbildungsmittel können z.B. Talkum, Calciumfluorid, Natriumphenylphosphinat, Aluminiumoxid, Siliciumdioxid und Nylon 22 enthalten sein.

Gleit- und Entformungsmittel, welche in der Regel in Mengen bis zu 1 Gew.-%, bezogen auf das Gesamtgewicht der Polymermasse (A), eingesetzt werden können, sind z.B. langkettige Fettsäuren wie Stearinsäure oder Behensäure, deren Salze (z.B. Ca- oder Zn-Stearat) oder Ester (z.B. Stearylstearat oder Pentaerythrittetrastearat) sowie Amidderivate (z.B. Ethylenbisstearylamid).

Weiterhin können Antiblockmittel auf mineralischer Basis in Mengen bis zu 0,1 Gew.-%, bezogen auf das Gesamtgewicht der Polymermasse (A), enthalten sein. Als Beispiele seien amorphe oder kristalline Kieselsäure, Calciumcarbonat oder Aluminiumsilikat genannt.

Als Verarbeitungshilfsmittel kann beispielsweise Mineralöl, vorzugsweise medizinisches Weißöl, in Mengen bis zu 5 Gew.-%, vorzugsweise bis zu 2 Gew.-%, insbesondere 0,1 bis 2 Gew.-%, bezogen auf das Gesamtgewicht der Polymermasse (A), enthalten sein.

Als Beispiele für Weichmacher seien Phthalsäuredioctylester, Phthalsäuredibenzylester, Phthalsäurebutylbenzylester, Kohlenwasserstofföle, N-(n-Butyl)benzolsulfonamid und o- und p-Tolylethylsulfonamid genannt.

Weiterhin können alle für die jeweiligen Thermoplaste bekannten nicht-halogenhaltigen Flammschutzmittel enthalten sein, insbesondere solche auf Basis von Phosphorverbindungen.

Des Weiteren können Feuchtigkeit und/oder weitere anorganische und/oder organische Fremdbestandteile, wie z.B. Nahrungsmittelrückstände, Zementreste oder Kleberreste, in der Polymermasse (A) enthalten sein.

Die erfindungsgemäß eingesetzte Polymermasse (A) kann gegebenenfalls in geeigneter Weise vorbehandelt werden, um beispielsweise anhaftende Verunreinigungen wie etwa Lebensmittelreste oder Schmutz, Feuchtigkeit und Fremdstoffe wie Metalle oder andere Stoffe und Verbundmaterialien zu entfernen.

Dies erfolgt vorteilhaft in einer Vorbehandlung, die einen oder mehrere der folgenden Schritte umfassen kann, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen. Gegebenenfalls können auch Polymermassen, die der Polymermasse (A) nicht entsprechen, durch ein solches Verfahren in eine erfindungsgemäß eingesetzte Polymermasse (A) umgewandelt werden.

Bevorzugt ist daher auch eine Ausführungsform des erfindungsgemäßen Verfahrens, bei der eine Polymermasse, die der Polymermasse (A) nicht entspricht, einer Vorbehandlung unterworfen wird, die einen oder mehrere der folgenden Schritte umfasst, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen, um so die Polymermasse (A) zu erhalten.

Bei der anorganischen basischen Verbindung (B) handelt es sich insbesondere um eine Verbindung, die mindestens ein aus der Gruppe K, Na, Ca, Ba, Mg, Sr, Al, Ti und Si ausgewähltes Element enthält. Anorganische basische Verbindungen (B) umfassend diese Elemente weisen üblicherweise eine akzeptable Reaktivität gegenüber der mindestens einen halogenhaltigen Verbindung (A3) oder ihren Zersetzungsprodukten auf.

Die anorganische basische Verbindung (B) kann ausgewählt sein aus der Gruppe bestehend aus Oxiden, Hydroxiden, Carbonaten oder eine Kombination aus Oxiden und Hydroxiden der oben genannten Elemente. Beispiele für anorganische basische Verbindungen (B) sind K₂O, Na₂O, NaOH, CaO, BaO, SrO, Ca(OH)₂, Ba(OH)₂, CaCO₃, MgO, Al₂O₃, SiO₂, TiO₂, Mg(OH)₂ und SrO. Bevorzugt ist die anorganische basische Verbindung ausgewählt aus der Gruppe bestehend aus Alkalimetalloxiden, Erdalkalimetalloxiden, Alkalimetallhydroxiden, Erdalkalimetallhydroxiden und Kombinationen davon. Besonders bevorzugt ist die anorganische basische Verbindung (B) ausgewählt aus der Gruppe bestehend aus MgO, CaO, SrO, BaO, K₂O und Kombinationen davon. Ganz besonders bevorzugt ist die anorganische basische Verbindung (B) ausgewählt aus der Gruppe bestehend aus CaO, BaO und Kombinationen davon, insbesondere BaO.

Ohne an eine bestimmte Theorie gebunden zu sein, wird angenommen, dass die Alkali- oder Erdalkaliverbindung mit dem in dem halogenhaltigen Flammschutzmittel vorliegenden Halogenatom reagiert und so eine stabile Halogenverbindung entsteht. Die Abtrennung solcher stabilen Halogenverbindungen kann über übliche physikalische Trennverfahren erfolgen. Im Fall der Bildung organischer Halogenverbindungen lassen diese sich ebenfalls aus dem Reaktionsgemisch abtrennen, beispielsweise durch Destillation.

Die anorganische basische Verbindung (B) kann im Verfahrensschritt a) auch separat von den übrigen Komponenten der erfindungsgemäßen Polymermasse (A) in die Reaktionszone (R) des Pyrolysereaktors (P) eingebracht werden, beispielsweise als Feststoff. In einer bevorzugten Ausführungsform wird die anorganische basische Verbindung (B) separat in die Reaktionszone (R) des Pyrolysereaktors (P) eingebracht und während des Verfahrens ganz oder teilweise kontinuierlich oder diskontinuierlich aus der Reaktionszone (R) entnommen und durch unverbrauchte anorganische Verbindungen (B) ganz oder teilweise ersetzt.

In einer besonderen Ausführungsform kann die anorganische Verbindung (B) ganz oder teilweise das Bettmaterial in einem Wirbelschichtreaktor bilden und in einem separaten Kreislauf geführt und regeneriert werden. In einer weiteren besonderen Ausführungsform wird die anorganische, basische Verbindung (B) in Verbindung mit anderen anorganischen Verbindungen in die Reaktionszone (R) eingebracht, beispielsweise als Oberflächenbeschichtung auf einem Bettmaterial. In einer besonderen Ausführungsform wird das Bettmaterial, welches ganz oder teilweise aus der anorganischen, basischen Verbindung (B) besteht, nach der Reaktion unter oxidierenden Bedingungen erhitzt und dadurch regeneriert.

In einer Ausführungsform wird das Gewichtsverhältnis der mindestens einen anorganischen basischen Verbindung (B) zu der mindestens einen halogenhaltigen Verbindung (A3) so gewählt, dass es in dem Bereich von 1:1 bis 10:1, bevorzugt in dem Bereich von 1,5:1 bis 5:1, besonders bevorzugt in dem Bereich von 2:1 bis 3:1 liegt.

Erfindungsgemäß ist in der Polymermasse (A) und in der anorganischen basischen Verbindung (B), besonders bevorzugt bei dem gesamten erfindungsgemäßen Verfahren, kein Antimonoxid (Sb₂O₃) anwesend. Ganz besonders bevorzugt sind bei dem erfindungsgemäßen Verfahren gar keine Antimon-Verbindungen anwesend. Wenn solche Antimon-Verbindungen anwesend sind, kann es vorteilhaft sein, diese vor Schritt a) aus der Polymermasse zu entfernen. Ohne sich an eine Theorie binden zu wollen, wird vermutet, dass die Anwesenheit von Antimonverbindungen, insbesondere Antimonoxid, bei der Bildung von Styrol-Monomeren während der Pyrolyse von Styrolpolymeren nachteilig sein kann.

Nach dem Einbringen der Polymermasse (A) und der anorganischen basischen Verbindung (B) in die Reaktionszone (R) des Pyrolysereaktors (P) in Schritt a) erfolgt im Schritt b) ein thermisches Spalten des in der Polymermasse (A) enthaltenen Styrol-haltigen Polymers (A1) in der Reaktionszone (R) des Pyrolysereaktors (P), um ein Produktgemisch (G) enthaltend Styrol-Monomere und weitere Komponenten, zu erhalten.

Das thermische Spalten erfolgt vorzugsweise bei einer Temperatur von 250 °C bis 1000 °C, bevorzugt von 300 °C bis 700 °C, besonders bevorzugt von 380 °C bis 650 °C, ganz besonders bevorzugt von 400 °C bis 600 °C. Die mittlere Verweilzeit (Z) der Polymermasse (A) in der Reaktionszone (R) des Pyrolysereaktors wird hierbei bevorzugt bei 0,01 s bis 21600 s, besonders bevorzugt bei 0,01 bis 3600 s, ganz besonders bevorzugt von 0,01 s bis 500 s, insbesondere bevorzugt bei 0,1 bis 5 s gehalten.

In einer besonderen Ausführungsform erfolgt das thermische Spalten vorzugsweise bei einem Druck von weniger als 1200 mbar, bevorzugt bei einem Druck von weniger als 1013 mbar, besonders bevorzugt bei einem Druck von weniger als 500 mbar, ganz besonders bevorzugt bei einem Druck von weniger als 300 mbar.

Während der Verweilzeit (Z) in der Reaktionszone (R) des Pyrolysereaktors (P) wird das Styrol-haltige Polymer (A1) und gegebenenfalls weitere Polymere (A2) der Polymerzusammensetzung (A) zumindest teilweise depolymerisiert, sowie die mindestens eine halogenhaltige Verbindung (A3) und gegebenenfalls weitere Komponenten (A4), die gegenüber den gewählten Bedingungen empfindlich sind, zumindest teilweise zersetzt, wobei die mindestens eine halogenhaltige Verbindung (A3) und gegebenenfalls ihre Zersetzungsprodukte mit der mindestens einen basischen anorganischen Verbindung (B) reagieren, um ein Produktgemisch (G) enthaltend Styrol-Monomere und andere Komponenten zu ergeben.

Das Produktgemisch (G), enthaltend Styrol-Monomere und andere Komponenten wird in Schritt c) aus der Reaktionszone (R) des Pyrolysereaktors (P) entnommen. Bevorzugt findet die Entnahme kontinuierlich statt. Besonders bevorzugt wird das Produktgemisch (G) in gasförmigem Zustand aus dem oberen Bereich der Reaktionszone (R) des Pyrolysereaktors (P) kontinuierlich entnommen. Hierbei kann die Entnahme des Produktgemisches (G) unter reduziertem Druck erfolgen. In einer weiteren bevorzugten Ausführungsform kann die Entnahme des Produktgemisches (G) beispielsweise durch Beförderung des Produktgemisches (G) aus der Reaktionszone (R) aufgrund des reaktionsbedingt erhöhten Drucks in der Reaktionszone (R) automatisch erfolgen.

Der Pyrolysereaktor kann eine Quensche enthalten oder mit einer Quensche verbunden sein. Unter Quensche wird in diesem Zusammenhang ein Bereich des Pyrolysereaktors verstanden, in dem die Depolymerisationsreaktion schnell abgestoppt wird, bevorzugt durch Abkühlen des heißen Gases umfassend das Produktgemisch (G). Die Quensche dient unter anderem zur Stabilisierung der Reaktionsprodukte und zur Verhinderung oder Verminderung einer unerwünschten Repolymerisation. Typische Quenschen kühlen das Produktgemisch (G) von der in der Reaktionszone (R) des Pyrolysereaktors (P) eingestellten Temperatur auf eine Temperatur von unter 250 °C innerhalb eines sehr kurzen Zeitraums ab, bevorzugt innerhalb von weniger als 10 Sekunden, besonders bevorzugt innerhalb von weniger als 5 Sekunden, besonders bevorzugt innerhalb von weniger als 1 Sekunde. Quenschen sind z.B. beschrieben in EP 1966291.

Das Produktgemisch (G) wird in Schritt d) nach der Entnahme aus der Reaktionszone (R) des Pyrolysereaktos (P) abgekühlt, wodurch eine Kondensation der Styrol-Monomere und weiterer Komponenten stattfindet und ein kondensiertes Produktgemisch (G') enthaltend Styrol-Monomere und weitere Komponenten erhalten wird. Das Produktgemisch (G) wird auf eine Temperatur abgekühlt, die unter dem Kondensationspunkt von Styrol-Monomeren liegt. Bevorzugt wird das Produktgemisch (G) auf eine Temperatur unter 70 °C, besonders bevorzugt unter 50 °C, ganz besonders bevorzugt unter 40 °C gekühlt. In einer bevorzugten Ausführungsform wird das Produktgemisch (G) auf eine Temperatur von -200 °C bis 70 °C gekühlt. In einer besonders bevorzugten Ausführungsform wird das Produktgemisch (G) auf eine Temperatur von -200 °C bis 40 °C gekühlt. In einer weiteren Ausführungsform wird das Produktgemisch (G) auf eine Temperatur von -5 °C bis 30 °C gekühlt.

In einer weiteren Ausführungsform wird das Produktgemisch (G) in einer Kondensationsvorrichtung mit mindestens zwei Temperaturzonen gekühlt und kondensiert. In diesem Fall wird das Produktgemisch (G) in einer ersten Temperaturzone auf eine Temperatur von -30 °C bis 50 °C, bevorzugt von -15 °C bis 30 °C, besonders bevorzugt von -5 °C bis 15 °C gekühlt und in einer weiteren Temperaturzone auf eine Temperatur von -200 °C bis -50 °C, bevorzugt von -200 °C bis -100 °C, besonders bevorzugt von -200 °C bis -150 °C gekühlt. In dieser Ausführungsform findet die Kondensation vorzugsweise unter reduziertem Druck statt.

Die Abkühlung kann auf eine beliebige bekannte Art und Weise erfolgen. Beispielsweise ist die Abkühlung an einer festen Oberfläche möglich, die durch Wasser oder Luft gekühlt wird. Ebenfalls ist die Abkühlung durch einen Wassernebel möglich, der direkt mit dem Produktgemisch (G) in Kontakt gebracht wird.

Bevorzugt findet die Abkühlung in einem Wassernebel statt. Dabei werden die kondensierbaren Bestandteile des Produktgemischs (G) entsprechend ihren Dampfdrücken kondensiert und zusammen mit dem Wasser aufgefangen. Bei der Kondensation wird ein kondensiertes Produktgemisch (G') enthaltend Styrol-Monomere und weitere Bestandteile erhalten. Im Falle der Abkühlung in einem Wassernebel wird das kondensierte Produktgemisch (G') als zwei-Phasen-System mit dem Kühlwasser erhalten. In diesem Fall wird das kondensierte Produktgemisch (G') als aufschwimmende organische Phase von der wässrigen Phase getrennt. Die wässrige Phase kann erneut gekühlt und als Wassernebel zum Abkühlen des Produktgemischs (G) eingesetzt werden.

Das kondensierte Produktgemisch (G') enthält üblicherweise mehr als 10 Gew.-%, bezogen auf das Gesamtgewicht des kondensierten Produktgemischs (G'), Styrol-Monomere und weniger als 90 Gew.-%, bezogen auf das Gesamtgewicht des kondensierten Produktgemischs (G'), der weiteren Bestandteile. Vorzugsweise enthält das kondensierte Produktgemisch (G') 10 bis 99 Gew.-%, bezogen auf das Gesamtgewicht des kondensierten Produktgemischs (G'), Styrol-Monomere und 1 bis 90 Gew.-%, bezogen auf das Gesamtgewicht des kondensierten Produktgemischs (G'), der weiteren Bestandteile, besonders vorzugsweise 50 bis 99 Gew.-%, bezogen auf das Gesamtgewicht des kondensierten Produktgemischs (G'), Styrol-Monomere und 1 bis 50 Gew.-%, bezogen auf das Gesamtgewicht des kondensierten Produktgemischs (G'), der weiteren Bestandteile, ganz besonders vorzugsweise 70 bis 98 Gew.-%, bezogen auf das Gesamtgewicht des kondensierten Produktgemischs (G'), Styrol-Monomere und 2 bis 30 Gew.-%, bezogen auf das Gesamtgewicht des kondensierten Produktgemischs (G'), der weiteren Bestandteile.

Ferner enthält das kondensierte Produktgemisch (G') üblicherweise mehr als 40 Gew.-%, bevorzugt mehr als 50 Gew.-%, besonders bevorzugt mehr als 55 Gew.-%, ganz besonders bevorzugt mehr als 60 Gew.-% an Styrol, bezogen auf das Gesamtgewicht der Wiederholungseinheiten (la), die von Styrol stammen, in dem mindestens einen Styrol-haltigen Polymer (A1).

Im weiteren Verlauf des Verfahrens wird in Schritt e) beispielsweise das kondensierte Produktgemisch (G') in eine Styrol-Monomer-haltige Fraktion und weitere Fraktionen getrennt. Diese Trennung kann auf beliebige bekannte Weise durchgeführt werden, die geeignet ist, Mischungen aus flüssigen Produkten und gegebenenfalls Feststoffen in die Bestandteile aufzutrennen.

Abhängig von den weiteren Bestandteilen neben Styrol-Monomeren können geeignete Methoden zum Trennen des kondensierten Produktgemischs (G') in eine Styrol-Monomer-haltige Fraktion und weitere Fraktionen beispielsweise Sedimentation, Zentrifugation, Filtration, Dekantieren, Destillation, Chromatographie, Kristallisation und Sublimation umfassen.

Sind in der kondensierten Produktmischung (G') Feststoffe enthalten, ist es vorteilhaft, vor der Trennung der flüssigen Bestandteile die Feststoffe abzutrennen. In diesem Fall ist es bevorzugt, den Feststoff durch Sedimentation, Zentrifugation, Filtration, Destillation, Sublimation und/oder Dekantieren, besonders bevorzugt durch Filtration, ganz besonders bevorzugt durch Filtration mittels Fest-Flüssig-Filter von den flüssigen Bestandteilen abzutrennen.

Die weitere Auftrennung der flüssigen Bestandteile in eine Styrol-Monomer-haltige Fraktion und weitere Fraktionen umfasst vorzugsweise mindestens einen Schritt der Destillation, wie zum Beispiel fraktionierende Destillation, mindestens einen Schritt der Chromatographie, wie zum Beispiel Säulenchromatographie, HPLC oder Flash-Chromatographie, und/oder mindestens einen Schritt der Kristallisation, wie zum Beispiel fraktionierende Kristallisation. Besonders vorzugsweise umfasst die Auftrennung der flüssigen Bestandteile mindestens einen Schritt der Destillation, ganz besonders vorzugsweise mindestens einen Schritt der fraktionierenden Destillation. In einer besonders bevorzugten Ausführungsform umfasst die Auftrennung der flüssigen Bestandteile in eine Styrol-Monomer-haltige Fraktion und weitere Fraktionen mindestens einen Schritt der fraktionierenden Destillation in einer oder mehreren Rektifikationskolonnen. Hierbei wird die Styrol-Monomer-haltige Fraktion als Styrol-Monomer-haltige Flüssigkeit gesammelt.

In einer bevorzugten Ausführungsform liegt der Anteil an halogenierten Verbindungen in der Styrol-Monomer-haltigen Flüssigkeit bei weniger als 1000 ppm, bevorzugt bei weniger als 500 ppm, besonders bevorzugt bei weniger als 100 ppm, bezogen auf die Styrol-Monomer-haltige Flüssigkeit.

Häufig ist es vorteilhaft, zumindest einen Teil der weiteren Fraktionen in die Reaktionszone (R) des Pyrolysereaktors (P) zurückzuführen. Dies ist insbesondere vorteilhaft, wenn ein Teil der weiteren Fraktionen Styrol-Oligomere wie z.B. Styrol-Dimere und Styrol-Trimere enthält. Dies ermöglicht eine insgesamt bessere Ausbeute an Styrol-Monomeren, da hierdurch auch die Depolymerisation der übrig gebliebenen Oligomere ermöglicht wird.

Aus diesem Grund wird vorzugsweise zumindest ein Teil der weiteren Fraktionen des kondensierten Produktgemischs (G'), die von der Styrol-Monomer-haltigen Fraktion abgetrennt wurden, in die Reaktionszone (R) des Pyrolysereaktors (P) zurückgeführt. Besonders bevorzugt ist hierbei eine kontinuierliche Rückführung der weiteren Fraktionen, wobei die Rückführung der Fraktionen, die schwerer sieden als die Styrol-Monomer-haltige Fraktion, bevorzugt ist, da diese den höchsten Anteil an Styrol-Oligomeren enthalten.

In einer bevorzugten Ausführungsform bestehen die weiteren Komponenten, die in die Reaktionszone (R) des Pyrolysereaktors (P) zurückgeführt werden, im Wesentlichen aus Styrol-Oligomeren, vorzugsweise aus Styrol-Dimeren und Styrol-Trimeren. In diesem Zusammenhang bedeutet "im Wesentlichen", dass die weiteren Komponenten, die in die Reaktionszone (R) des Pyrolysereaktors (P) zurückgeführt werden, neben Styrol-Oligomeren keine weiteren Komponenten enthalten, die den Depolymerisationsprozess in der Reaktionszone (R) des Pyrolysereaktors (P) stören.

Geeignet zur Durchführung des Verfahrens gemäß der Erfindung ist eine Vorrichtung zur Durchführung eines Verfahrens zur Herstellung von Styrol-Monomeren aus einer Styrol-haltigen Polymermasse wie oben beschrieben, bei der die Reaktionszone (R) und der Pyrolysereaktor (P) in der Vorrichtung so gestaltet sind, dass eine schonende Depolymerisation der Polymermasse (A) erfolgen kann.

Vorzugsweise umfasst die Vorrichtung einen Pyrolysereaktor (P) mit einer Reaktionszone (R), mindestens ein Heizelement zum Einstellen der gewünschten Temperaturen, mindestens eine Leitung zum Einführen der Polymermasse (A) in die Reaktionszone (R) und zum Einstellen der Verweilzeit (Z), sowie eine Quensche zum Abkühlen des Produktgemischs (G).

Ein weiterer Gegenstand der vorliegenden Erfindung ist das kondensierte Produktgemisch (G'), enthaltend Styrol-Monomere und weitere Komponenten, welches aus Schritt d) des erfindungsgemäßen Verfahrens erhältlich ist.

Die Styrol-Monomer-haltige Flüssigkeit, die durch das erfindungsgemäße Verfahren erhältlich ist, ist ebenfalls Gegenstand der Erfindung. Vorzugsweise enthält diese Flüssigkeit weniger als 1000 ppm, bevorzugt weniger als 500 ppm, besonders bevorzugt weniger als 100 ppm, bezogen auf das Gesamtgewicht der Styrol-Monomer-haltigen Flüssigkeit, an halogenierten Verbindungen.

Ein weiterer Gegenstand der Erfindung ist die Verwendung einer anorganischen basischen Verbindung (B), wie oben beschrieben, für den Abbau halogenhaltiger organischer Verbindungen bei der pyrolytischen Rückgewinnung von Monomeren aus Polymermassen in Abwesenheit von Antimonoxid (SbO₃), vorzugsweise aus Polymermassen umfassend Styrol-haltige Polymere.

Die Erfindung wird durch die folgenden Beispiele und Ansprüche illustriert.

Die die Erfindung betreffenden Experimente wurden im Batchverfahren im Labormaßstab durchgeführt. Hierzu wurde als Batchreaktor ein modifizierter Labor-Rührautoklav Versoclave Typ 3E/2,0 It. der Firma Büchi AG (Uster, Schweiz) eingesetzt, welcher über einen elektrischen Heizmantel bis 500 °C beheizt werden kann. Die Temperaturkontrolle erfolgte über Temperaturfühler im Mantel, im Reaktorboden sowie einen über die Deckelplatte eingeführten Tauchfühler in der Schmelze. Das vorgelegte Material wurde während des Depolymerisationsprozesses über ein Rührwerk mit einem Anker-Rührer durchmischt. Über einen Auslassstutzen in der Deckelplatte des Reaktors wurde das flüchtige Produktgemisch in einer Glaskondensationsvorrichtung mit zwei Temperaturzonen (0 °C und -196 °C) kondensiert und aufgefangen. Die gesamte Versuchsapparatur bestehend aus Batchreaktor und Kondensationsvorrichtung wurde über eine nachgeschaltete Membranpumpe evakuiert.

Für Vergleichsversuch 2 und Versuche 1 bis 3 wurden 98 Teile PS, zwei Teile HBCD und, zusätzlich bei den Versuchen 1 bis 3, fünf Teile einer anorganischen Base eingesetzt. Als anorganische Base wurde in den Versuchen MgO (Versuch 1), CaO (Versuch 2) und BaO (Versuch 3) eingesetzt. Typische Ansatzmengen sind 245 g PS, 5 g HBCD und 12,5 g der anorganischen Base. Nach Bestückung mit dem Reaktionsgemisch wurde der Autoklav verschlossen, evakuiert und über mehrere Stunden (typischerweise 5 bis 6 Stunden) unter Rühren sowie in mehreren Stufen bis zu einer Zieltemperatur von 380 °C (gemessen am Reaktorboden) erhitzt. Während des Erhitzens setzte die Depolymerisation des Reaktionsgemisches ein.

Die flüchtigen Reaktionsprodukte wurden wie oben beschrieben fraktioniert kondensiert. Die so erhaltenen Fraktionen wurden mittels Gaschromatographie und Elementaranalyse zur Bestimmung des Styrol- und Halogengehaltes charakterisiert. Die gaschromatographischen Untersuchungen wurden mit einem Gaschromatographen vom Typ Agilent 7890a mit einer Säule DB-1, Länge 25 m (Agilent) durchgeführt. Die Detektion erfolgte mittels FID-Detektor und als Lösungsmittel wurde THF eingesetzt.

Für den Vergleichsversuch 1 wurde PS im Batchprozess depolymerisiert. Die Ansatzmenge war 500 g PS. Nach Bestückung mit dem Reaktionsgemisch wurde der Autoklav verschlossen, evakuiert und über mehrere Stunden (5 Stunden) unter Rühren sowie in mehreren Stufen bis 380 °C (gemessen am Reaktorboden) erhitzt. Gegen Ende des Versuches wurde die Temperatur für 10 Minuten bis auf 450 °C (gemessen am Reaktorboden) gesteigert. Während des Erhitzens setzte die Depolymerisation des Reaktionsgemisches ein. Die flüchtigen Produkte wurden wie oben beschrieben fraktioniert kondensiert. Die so erhaltenen Fraktionen wurden vereint und mittels Gaschromatographie charakterisiert.

Die Ergebnisse sind in Tabelle 1 dargestellt. Die angegebene Kondensat-Ausbeute ist die Masse der flüchtigen Verbindungen aller aufgefangenen Fraktionen in Relation zur Summe der eingesetzten Masse des Reaktionsgutes. Die Styrol-Ausbeute bezieht sich auf die Masse des Styrols im Reaktionsgut. Der Bromgehalt ist der prozentuale Massegehalt von Brom im gesamten Kondensat. Die Abfangeffizienz errechnet sich aus der Relation von detektiertem Bromgehalt im gesamten Kondensat zum Bromgehalt im eingesetzten Reaktionsgut. Dabei beträgt der Massegehalt von Brom in HBCD gerade 74,71 Gew.-%.

**Tabelle 1: Vergleich der durchgeführten Experimente**

| | Vergleichsversuch 1 | Vergleichsversuch 2 | Versuch 1 | Versuch 2 | Versuch 3 |
|---|---|---|---|---|---|
| Anteil PS [Gew.-Teile] | 100 | 98 | 98 | 98 | 98 |
| Anteil HBCD [Gew.-Teile] | 0 | 2 | 2 | 2 | 2 |
| Anteil Base [Gew.-Teile] | 0 | 0 | 5 | 5 | 5 |
| Eingesetzte Base | - | - | MgO | CaO | BaO |
| Kondensatausbeute [Gew.-%] | 97,68 | 89,13 | 91,84 | 95,44 | 92,88 |
| Styrolausbeute [Gew.-%] | 58,12 | 43,33 | 44,70 | 60,50 | 61,02 |
| Bromgehalt im Kondensat [g] | - | 2,060 | 1,945 | 0,153 | 0,158 |
| Bromgehalt im Edukt [g] | - | 3,735 | 3,735 | 3,735 | 3,735 |
| Abfangeffizienz [Gew.-%] | - | 44,8 | 47,9 | 95,9 | 95,8 |

Anhand der Messergebnisse ist deutlich zu erkennen, dass der Zusatz von anorganischen Basen, insbesondere der Komponenten CaO und/oder BaO, zu einer deutlichen Reduktion des Bromgehalts im Kondensat führt. Auch ist die Styrol-Ausbeute gut.

Das beschriebene Verfahren kann auch mehrfach nacheinander durchgeführt werden und gegebenenfalls zu einer weiteren Abreicherung an halogenhaltigen Verbindungen führen.

HBCD-haltige Polymermassen ergeben eine geringere Ausbeute an Styrol-Öl, wie ein Vergleich der Ausbeuten von Vergleichsversuch 1 und Vergleichsversuch 2 zeigt. Der Zusatz von anorganischen Basen gleicht die Ausbeuteverluste bei der Depolymerisation von flammschutzmittelhaltigen Polymermassen aus oder überkompensiert die Ausbeuteverluste sogar, wie anhand der Versuche 1 bis 3, insbesondere anhand der Versuche 2 und 3, deutlich zu erkennen ist.

Analoge Experimente sind auch mit anderen Styrol-haltigen Polymeren (als Polystyrol) durchführbar.

## Patentansprüche

1. Verfahren zur Rückgewinnung von Styrol-Monomeren aus einer Polymermasse (A) durch Pyrolyse unter Abbau der darin enthaltenen halogenhaltigen Verbindungen, umfassend die Schritte:
a) Einbringen von:
I) 25 bis 99,95 Gew.-%, bezogen auf das Gesamtgewicht von (A) und (B), einer Polymermasse (A) umfassend
A1) 30 bis 99,9999 Gew.-%, bezogen auf das Gesamtgewicht der Polymermasse (A), mindestens eines Styrol-haltigen Polymers (A1) umfassend
la) 10 bis 100 Gew.-%, bezogen auf das Gesamtgewicht des Styrol-haltigen Polymers (A1), an Wiederholungseinheiten (la), die von Styrol stammen; und
lb) 0 bis 90 Gew.-%, bezogen auf das Gesamtgewicht des Styrol-haltigen Polymers (A1), an Kautschuk und/oder an Wiederholungseinheiten (Ib), die sich von den Wiederholungseinheiten (la) unterscheiden und den Pyrolyseprozess nicht unterbrechen, bevorzugt ausgewählt aus Wiederholungseinheiten (Ib), die von Acrylnitril, Acrylestern, Methylmethacrylat oder alpha-Methylstyrol stammen, oder Kombinationen davon;
A2) 0 bis 69,9999 Gew.-%, bezogen auf das Gesamtgewicht der Polymermasse (A), weiterer Polymere (A2), die sich von dem mindestens einen Styrol-haltigen Polymer (A1) unterscheiden und den Pyrolyseprozess nicht unterbrechen, bevorzugt ausgewählt aus der Gruppe bestehend aus Polyolefinen, Polycarbonaten, Polyestern, Polyamiden, Polyalkyl(meth)acrylaten, Polyurethanen und Kombinationen davon, besonders bevorzugt ausgewählt aus der Gruppe bestehend aus Polyethylen, Polypropylen, Polymethylmethacrylat und Kombinationen davon, besonders bevorzugt Polymethylmethacrylat;
A3) 0,0001 bis 50 Gew.-%, bezogen auf das Gesamtgewicht der Polymermasse (A), mindestens einer halogenhaltigen Verbindung (A3); und
A4) 0 bis 50 Gew.-%, bezogen auf das Gesamtgewicht der Polymermasse (A), weiterer Komponenten (A4), die den Pyrolyseprozess nicht unterbrechen, bevorzugt ausgewählt aus der Gruppe bestehend aus Füllstoffen, Stabilisatoren, anderen organischen und/oder anorganischen Additiven, Feuchtigkeit und anderen organischen und/oder anorganischen Fremdstoffen; und
II)0,05 bis 75 Gew.-%, bezogen auf das Gesamtgewicht von (A) und (B), mindestens einer anorganischen basischen Verbindung (B),
in die Reaktionszone (R) eines Pyrolysereaktors (P);
b) thermisches Spalten des in der Polymermasse (A) enthaltenen Styrol-haltigen Polymers (A1) in der Reaktionszone (R) des Pyrolysereaktors (P), um ein Produktgemisch (G), enthaltend Styrol-Monomere und weitere Komponenten, zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (G'), enthaltend Styrol-Monomere und weitere Komponenten, zu erhalten; und
e) Abtrennen einer Styrol-Monomer-haltigen Fraktion von den weiteren Komponenten des in Schritt d) erhaltenen kondensierten Produktgemisches (G'), um eine Styrol-Monomer-haltige Flüssigkeit zu erhalten,
**dadurch gekennzeichnet, dass** in der Polymermasse (A) und in der anorganischen basischen Verbindung (B), kein Antimonoxid (Sb₂O₃) vorhanden ist.

2. Verfahren gemäß Anspruch 1, **dadurch gekennzeichnet, dass** der Verfahrensschritt b) bei einer Temperatur von 250 °C bis 1000 °C, bevorzugt von 300 °C bis 700 °C, besonders bevorzugt von 380 °C bis 650 °C, ganz besonders bevorzugt von 400 °C bis 600 °C erfolgt.

3. Verfahren gemäß Anspruch 1 oder 2, wobei die mittlere Verweilzeit (Z) der Polymermasse (A) in der Reaktionszone (R) des Pyrolysereaktors (P) in Schritt b) von 0,01 s bis 21600 s, bevorzugt von 0,01 s bis 3600 s, besonders bevorzugt von 0,01 s bis 500 s, ganz besonders bevorzugt von 0,1 bis 5 s beträgt.

4. Verfahren gemäß mindestens einem der Ansprüche 1 bis 3, **dadurch gekennzeichnet, dass** der Verfahrensschritt b) bei einem Druck von weniger als 1200 mbar, bevorzugt bei einem Druck von weniger als 1013 mbar, besonders bevorzugt bei einem Druck von weniger als 500 mbar, ganz besonders bevorzugt bei einem Druck von weniger als 300 mbar erfolgt.

5. Verfahren gemäß mindestens einem der Ansprüche 1 bis 4, **dadurch gekennzeichnet, dass** das Abtrennen der Styrol-Monomer-haltigen Fraktion in Schritt e) durch fraktionierende Destillation erfolgt, bevorzugt mittels mindestens einer Rektifikationskolonne, besonders bevorzugt mittels mindestens zweier Rektifikationskolonnen.

6. Verfahren gemäß mindestens einem der Ansprüche 1 bis 5, **dadurch gekennzeichnet, dass** die mindestens eine halogenhaltige Verbindung (A3) mindestens ein organisches halogenhaltiges Flammschutzmittel ist, bevorzugt ein bromhaltiges Flammschutzmittel, besonders bevorzugt Hexabromcyclododecan (HBCD) und/oder polybromiertes Styrol-Butadien-Copolymer.

7. Verfahren gemäß mindestens einem der Ansprüche 1 bis 6, **dadurch gekennzeichnet, dass** die Polymermasse (A) mindestens 10 ppm, bevorzugt mindestens 100 ppm, besonders bevorzugt mindestens 1000 ppm der mindestens einen, halogenhaltigen Verbindung (A3) enthält.

8. Verfahren gemäß mindestens einem der Ansprüche 1 bis 7, **dadurch gekennzeichnet, dass** die mindestens eine anorganische basische Verbindung (B) ausgewählt ist aus der Gruppe bestehend aus basischen Verbindungen der Haupt- oder Nebengruppenmetalle, vorzugsweise aus der Gruppe bestehend aus basischen Alkali- und/oder Erdalkalimetallverbindungen.

9. Verfahren gemäß einem mindestens der Ansprüche 1 bis 8, **dadurch gekennzeichnet, dass** die mindestens eine anorganische basische Verbindung (B) ausgewählt ist aus der Gruppe bestehend aus Alkalimetalloxiden, Erdalkalimetalloxiden, Alkalimetallhydroxiden, Erdalkalimetallhydroxiden und Kombinationen davon, bevorzugt aus der Gruppe bestehend aus MgO, CaO, SrO, BaO, K₂O und Kombinationen davon, besonders bevorzugt aus der Gruppe bestehend aus CaO, BaO und Kombinationen davon, insbesondere BaO.

10. Verfahren gemäß mindestens einem der Ansprüche 1 bis 9, **dadurch gekennzeichnet, dass** das Styrol-haltige Polymer (A1) Polystyrol ist und gegebenenfalls die weiteren Polymere (A2) Polymethylmethacrylat sind.

11. Verfahren gemäß mindestens einem der Ansprüche 1 bis 10, **dadurch gekennzeichnet, dass** die mindestens eine anorganische basische Verbindung (B) unabhängig von der Polymermasse (A) in die Reaktionszone (R) des Pyrolysereaktors (P) eingebracht und während des Verfahrens ganz oder teilweise kontinuierlich oder diskontinuierlich aus der Reaktionszone (R) entnommen und durch unverbrauchte anorganische basische Verbindungen (B) ganz oder teilweise ersetzt wird.

12. Verfahren gemäß mindestens einem der Ansprüche 1 bis 11, **dadurch gekennzeichnet, dass** der Pyrolysereaktor (P) ein Wirbelschichtreaktor ist, und die Wirbelschicht ganz oder teilweise aus der mindestens einen anorganischen basischen Verbindung (B) besteht.

13. Verfahren gemäß mindestens einem der Ansprüche 1 bis 11, **dadurch gekennzeichnet, dass** der Pyrolysereaktor (P) ein Doppelschneckenextruder oder ein kontinuierlicher Rührtankreaktor oder ein Vortex-Reaktor ist.

14. Verfahren gemäß mindestens einem der Ansprüche 1 bis 13, **dadurch gekennzeichnet, dass** die Reaktionszone (R) des Pyrolysereaktors (P) durch eine Mikrowellen-unterstützte Heizung beheizt wird.

15. Verfahren gemäß mindestens einem der Ansprüche 1 bis 14, **dadurch gekennzeichnet, dass** bei dem Verfahren keine Antimon-Verbindungen anwesend sind.

16. Verfahren gemäß mindestens einem der Ansprüche 1 bis 15, **dadurch gekennzeichnet, dass** das kondensierte Produktgemisch (G') mehr als 40 Gew.-%, bevorzugt mehr als 50 Gew.-%, besonders bevorzugt mehr als 55 Gew.-%, ganz besonders bevorzugt mehr als 60 Gew.-% an Styrol enthält, bezogen auf das Gesamtgewicht der Wiederholungseinheiten (la), die von Styrol stammen, in dem mindestens einen Styrol-haltigen Polymer (A1).

17. Kondensiertes Produktgemisch (G') enthaltend Styrol-Monomere und weitere Komponenten, erhältlich aus Schritt d) des Verfahrens gemäß mindestens einem der Ansprüche 1 bis 16.

18. Styrol-Monomer-haltige Flüssigkeit erhältlich durch das Verfahren gemäß mindestens einem der Ansprüche 1 bis 16.

19. Styrol-Monomer-haltige Flüssigkeit gemäß Anspruch 18, **dadurch gekennzeichnet, dass** der Anteil an halogenierten Verbindungen weniger als 1000 ppm, bevorzugt bei weniger als 500 ppm, besonders bevorzugt bei weniger als 100 ppm, bezogen auf das Gesamtgewicht der Styrol-Monomer-haltigen Flüssigkeit, beträgt.

20. Verwendung einer anorganischen basischen Verbindung (B), wie in mindestens einem der Ansprüche 1 bis 16 definiert, für den Abbau halogenhaltiger organischer Verbindungen bei der pyrolytischen Rückgewinnung von Monomeren aus Polymermassen in Abwesenheit von Antimonoxid (SbO₃), vorzugsweise aus Polymermassen umfassend Styrol-haltige Polymere.

## Claims

1. A process for recovering styrene monomers from a polymer composition (A) by pyrolysis with decomposition of the halogen-containing compounds present therein, comprising the steps of:
a) introducing:
I) 25% to 99.95% by weight, based on the total weight of (A) and (B), of a polymer composition (A) comprising
A1) 30% to 99.9999% by weight, based on the total weight of the polymer composition (A), of at least one styrene-containing polymer (A1) comprising
Ia) 10% to 100% by weight, based on the total weight of the styrene-containing polymer (A1), of repeating units (Ia) deriving from styrene; and
Ib) 0% to 90% by weight, based on the total weight of the styrene-containing polymer (A1), of rubber and/or of repeating units (Ib) which are distinct from the repeating units (Ia) and do not interrupt the pyrolysis process, preferably selected from repeating units (Ib) derived from acrylonitrile, acrylic esters, methyl methacrylate or alpha-methylstyrene or combinations thereof;
A2) 0% to 69.9999% by weight, based on the total weight of the polymer composition (A), of further polymers (A2) which are distinct from the at least one styrene-containing polymer (A1) and do not interrupt the pyrolysis process, preferably selected from the group consisting of polyolefins, polycarbonates, polyesters, polyamides, polyalkyl (meth)acrylates, polyurethanes and combinations thereof, particularly preferably selected from the group consisting of polyethylene, polypropylene, polymethyl methacrylate and combinations thereof, particularly preferably polymethyl methacrylate;
A3) 0.0001% to 50% by weight, based on the total weight of the polymer composition (A), of at least one halogen-containing compound (A3); and
A4) 0% to 50% by weight, based on the total weight of the polymer composition (A), of further components (A4) which do not interrupt the pyrolysis process, preferably selected from the group consisting of fillers, stabilizers, other organic and/or inorganic additives, moisture and other organic and/or inorganic impurities; and
II) 0.05% to 75% by weight, based on the total weight of (A) and (B), of at least one inorganic basic compound (B),
into the reaction zone (R) of a pyrolysis reactor (P);
b) thermally cleaving the styrene-containing polymer (A1) present in the polymer composition (A) in the reaction zone (R) of the pyrolysis reactor (P) to obtain a product mixture (G) containing styrene monomers and further components;
c) withdrawing the product mixture (G) obtained in step b) from the reaction zone (R) of the pyrolysis reactor (P);
d) cooling the product mixture (G) withdrawn in step c) to obtain a condensed product mixture (G') containing styrene monomers and further components; and
e) separating a styrene monomer-containing fraction from the further components of the condensed product mixture (G') obtained in step d) to obtain a styrene monomer-containing liquid,
**characterized in that** no antimony oxide (Sb₂O₃) is present in the polymer composition (A) or in the inorganic basic compound (B).

2. The process as claimed in claim 1, **characterized in that** process step b) is carried out at a temperature of 250°C to 1000°C, preferably of 300°C to 700°C, particularly preferably of 380°C to 650°C, very particularly preferably of 400 °C to 600 °C.

3. The process as claimed in claim 1 or 2, wherein the average residence time (Z) of the polymer composition (A) in the reaction zone (R) of the pyrolysis reactor (P) in step b) is from 0.01 seconds to 21 600 s, preferably from 0.01 to 3600 s, particularly preferably from 0.01 to 500 seconds, very particularly preferably from 0.1 to 5 s.

4. The process as claimed in any of claims 1 to 3, **characterized in that** process step b) is carried out at a pressure of less than 1200 mbar, preferably at a pressure of less than 1013 mbar, particularly preferably at a pressure of less than 500 mbar, very particularly preferably at a pressure of less than 300 mbar.

5. The process as claimed in any of claims 1 to 4, **characterized in that** the separating of the styrene monomer-containing fraction in step e) is carried out by fractional distillation, preferably using at least one rectification column, particularly preferably using at least two rectification columns.

6. The process as claimed in at least one of claims 1 to 5, **characterized in that** the at least one halogen-containing compound (A3) is at least one organic halogen-containing flame retardant, preferably a bromine-containing flame retardant, particularly preferably hexabromocyclododecane (HBCD) and/or polybrominated styrene-butadiene copolymer.

7. The process as claimed in at least one of claims 1 to 6, **characterized in that** the polymer composition (A) contains at least 10 ppm, preferably at least 100 ppm, particularly preferably at least 1000 ppm, of the at least one halogen-containing compound (A3).

8. The process as claimed in at least one of claims 1 to 7, **characterized in that** the at least one inorganic basic compound (B) is selected from the group consisting of basic compounds of main group or transition group metals, preferably from the group consisting of basic alkali metal and/or alkaline earth metal compounds.

9. The process as claimed in any of claims 1 to 8, **characterized in that** the at least one inorganic basic compound (B) is selected from the group consisting of alkali metal oxides, alkaline earth metal oxides, alkali metal hydroxides, alkaline earth metal hydroxides and combinations thereof, preferably from the group consisting of MgO, CaO, SrO, BaO, K₂O and combinations thereof, particularly preferably from the group consisting of CaO, BaO and combinations thereof, in particular BaO.

10. The process as claimed in at least one of claims 1 to 9, **characterized in that** the styrene-containing polymer (A1) is polystyrene and any further polymers (A2) present are polymethyl methacrylate.

11. The process as claimed in at least one of claims 1 to 10, **characterized in that** the at least one inorganic basic compound (B) is introduced into the reaction zone (R) of the pyrolysis reactor (P) independently of the polymer composition (A) and in the course of the process wholly or partially continuously or discontinuously withdrawn from the reaction zone (R) and wholly or partially replaced by unused inorganic basic compounds (B).

12. The process as claimed in at least one of claims 1 to 11, **characterized in that** the pyrolysis reactor (P) is a fluidized bed reactor and the fluidized bed wholly or partially consists of the at least one inorganic basic compound (B).

13. The process as claimed in at least one of claims 1 to 11, **characterized in that** the pyrolysis reactor (P) is a twin-screw extruder or a continuous stirred tank reactor or a vortex reactor.

14. The process as claimed in at least one of claims 1 to 13, **characterized in that** the reaction zone (R) of the pyrolysis reactor (P) is heated through microwave-assisted heating.

15. The process as claimed in at least one of claims 1 to 14, **characterized in that** no antimony compounds are present in the process.

16. The process as claimed in at least one of claims 1 to 15, **characterized in that** the condensed product mixture (G') contains more than 40% by weight, preferably more than 50% by weight, particularly preferably more than 55% by weight, very particularly preferably more than 60% by weight, of styrene based on the total weight of the repeating units (Ia) derived from styrene in the at least one styrene-containing polymer (A1).

17. A condensed product mixture (G') containing styrene monomers and further components obtainable from step d) of the process as claimed in at least one of claims 1 to 16.

18. A styrene monomer-containing liquid obtainable by the process as claimed in at least one of claims 1 to 16.

19. The styrene monomer-containing liquid as claimed in claim 18, **characterized in that** the proportion of halogenated compounds is less than 1000 ppm, preferably less than 500 ppm, particularly preferably less than 100 ppm, based on the total weight of the styrene monomer-containing liquid.

20. The use of an inorganic basic compound (B) as defined in at least one of claims 1 to 16 for decomposition of halogen-containing organic compounds in the pyrolytic recovery of monomers from polymer compositions in the absence of antimony oxide (SbO₃), preferably from polymer compositions comprising styrene-containing polymers.

## Revendications

1. Procédé de récupération de monomères de styrène à partir d'une masse polymère (A) par pyrolyse, avec dégradation des composés halogénés qu'elle contient, comprenant les étapes suivantes :
a) Introduction de :
I) 25 à 99,95 % en poids, rapportés au poids total de (A) et (B), d'une masse polymère (A) comprenant
A1) 30 à 99,9999 % en poids, rapportés au poids total de la masse polymère (A), d'au moins un polymère contenant du styrène (A1) comprenant
Ia) 10 à 100 % en poids, rapportés au poids total du polymère contenant du styrène (A1), d'unités répétitives (Ia) dérivées du styrène ; et
Ib) 0 à 90 % en poids, rapportés au poids total du polymère contenant du styrène (A1), de caoutchouc et/ou d'unités répétitives (Ib) qui se distinguent des unités répétitives (Ia) et qui n'interrompent pas le processus de pyrolyse, de préférence choisies parmi les unités répétitives (Ib) dérivées de l'acrylonitrile, d'esters acryliques, du méthacrylate de méthyle ou de l'alpha-méthylstyrène, ou de combinaisons de ceux-ci ;
A2) 0 à 69,9999 % en poids, rapportés au poids total de la masse polymère (A), d'autres polymères (A2) qui diffèrent de l'au moins un polymère contenant du styrène (A1) et qui n'interrompent pas le processus de pyrolyse, de préférence choisis parmi le groupe constitué de polyoléfines, de polycarbonates, de polyesters, de polyamides, de polyalkyl(méth)acrylates, de polyuréthanes et de leurs combinaisons, de préférence choisis parmi le groupe constitué du polyéthylène, du polypropylène, du polyméthacrylate de méthyle et de leurs combinaisons, et plus particulièrement du polyméthacrylate de méthyle ;
A3) 0,0001 à 50 % en poids, rapportés au poids total de la masse polymère (A), d'au moins un composé halogéné (A3) ; et
A4) 0 à 50 % en poids, rapportés au poids total de la masse polymère (A), d'autres composants (A4) qui n'interrompent pas le processus de pyrolyse, choisis de préférence parmi le groupe constitué de charges, de stabilisants, d'autres additifs organiques et/ou inorganiques, d'humidité et d'autres substances étrangères organiques et/ou inorganiques ; et
II) 0,05 à 75 % en poids, rapportés au poids total de (A) et (B), d'au moins un composé basique inorganique (B),
dans la zone de réaction (R) d'un réacteur de pyrolyse (P) ;
b) craquage thermique du polymère contenant du styrène (A1), présent dans la masse polymère (A), dans la zone de réaction (R) du réacteur de pyrolyse (P) afin d'obtenir un mélange de produits (G) contenant des monomères de styrène et d'autres composants ;
c) prélèvement du mélange de produits (G) obtenu à l'étape b) de la zone de réaction (R) du réacteur de pyrolyse (P) ;
d) refroidissement du mélange de produits (G) prélevé à l'étape c) afin d'obtenir un mélange de produits condensé (G') contenant des monomères de styrène et d'autres composants ; et
e) séparation d'une fraction contenant des monomères de styrène des autres composants du mélange de produits condensés (G') obtenu à l'étape d) afin d'obtenir un liquide contenant des monomères de styrène,
**caractérisé en ce que** la masse polymère (A) et le composé basique inorganique (B) ne contiennent pas d'oxyde d'antimoine (Sb₂O₃).

2. Procédé selon la revendication 1, **caractérisé en ce que** l'étape b) est réalisée à une température comprise entre 250 °C et 1 000 °C, de préférence entre 300 °C et 700 °C, particulièrement 380 °C et 650 °C, et plus particulièrement entre 400 °C et 600 °C.

3. Procédé selon la revendication 1 ou 2, dans lequel le temps de séjour moyen (Z) de la masse polymère (A) dans la zone de réaction (R) du réacteur de pyrolyse (P) à l'étape b) est comprise entre 0,01 s et 21 600 s, de préférence entre 0,01 s et 3 600 s, particulièrement entre 0,01 s et 500 s, et plus particulièrement entre 0,1 s et 5 s.

4. Procédé selon au moins l'une des revendications 1 à 3, **caractérisé en ce que** l'étape b) du procédé est réalisée à une pression inférieure à 1 200 mbar, de préférence à une pression inférieure à 1 013 mbar, particulièrement à une pression inférieure à 500 mbar, et plus particulièrement à une pression inférieure à 300 mbar.

5. Procédé selon au moins l'une des revendications 1 à 4, **caractérisé en ce que** la séparation de la fraction contenant des monomères de styrène à l'étape e) s'effectue par distillation fractionnée, de préférence à l'aide d'au moins une colonne de rectification, et particulièrement à l'aide d'au moins deux colonnes de rectification.

6. Procédé selon au moins l'une des revendications 1 à 5, **caractérisé en ce que** l'au moins un composé halogéné (A3) est au moins un retardateur de flamme organique halogéné, de préférence un retardateur de flamme contenant du brome, et particulièrement l'hexabromocyclododécane (HBCD) et/ou un copolymère styrène-butadiène polybromé.

7. Procédé selon au moins l'une des revendications 1 à 6, **caractérisé en ce que** la masse polymère (A) contient au moins 10 ppm, de préférence au moins 100 ppm, particulièrement au moins 1 000 ppm, dudit au moins un composé halogéné (A3).

8. Procédé selon au moins l'une des revendications 1 à 7, **caractérisé en ce que** l'au moins un composé basique inorganique (B) est choisi parmi le groupe constitué de composés basiques des métaux du groupe principal ou des métaux du groupe secondaire, de préférence parmi le groupe constitué de composés basiques des métaux alcalins et/ou alcalino-terreux.

9. Procédé selon au moins l'une des revendications 1 à 8, **caractérisé en ce que** l'au moins un composé basique inorganique (B) est choisi parmi le groupe constitué d'oxydes de métaux alcalins, d'oxydes de métaux alcalino-terreux, d'hydroxydes de métaux alcalins, d'hydroxydes de métaux alcalino-terreux et de leurs combinaisons, de préférence parmi le groupe constitué de MgO, CaO, SrO, BaO, K₂O et de leurs combinaisons, de préférence parmi le groupe constitué de CaO, BaO et de leurs combinaisons, en particulier BaO.

10. Procédé selon au moins l'une des revendications 1 à 9, **caractérisé en ce que** le polymère (A1) contenant du styrène est du polystyrène et, le cas échéant, les autres polymères (A2) sont du polyméthacrylate de méthyle.

11. Procédé selon au moins l'une des revendications 1 à 10, **caractérisé en ce que** l'au moins un composé basique inorganique (B) est introduit dans la zone de réaction (R) du réacteur de pyrolyse (P), indépendamment de la masse polymère (A) et, au cours du procédé, est retiré en tout ou en partie, de manière continue ou discontinue, de la zone de réaction (R) et remplacé en tout ou en partie par des composés basiques inorganiques (B) non consommés.

12. Procédé selon au moins l'une des revendications 1 à 11, **caractérisé en ce que** le réacteur de pyrolyse (P) est un réacteur à lit fluidisé et le lit fluidisé est constitué, en totalité ou en partie, de l'au moins un composé basique inorganique (B).

13. Procédé selon au moins l'une des revendications 1 à 11, **caractérisé en ce que** le réacteur de pyrolyse (P) est une extrudeuse à double vis, un réacteur à cuve agitée en continu ou un réacteur à vortex.

14. Procédé selon au moins l'une des revendications 1 à 13, **caractérisé en ce que** la zone de réaction (R) du réacteur de pyrolyse (P) est chauffée par un système de chauffage à micro-ondes.

15. Procédé selon au moins l'une des revendications 1 à 14, **caractérisé en ce qu'**aucun composé d'antimoine n'est présent dans le procédé.

16. Procédé selon au moins l'une des revendications 1 à 15, **caractérisé en ce que** le mélange de produits condensés (G') contient plus de 40 % en poids, de préférence plus de 50 % en poids, particulièrement plus de 55 % en poids, et plus particulièrement plus de 60 % en poids de styrène, par rapport au poids total des unités répétitives (Ia) dérivées du styrène, dans l'au moins un polymère contenant du styrène (A1).

17. Mélange de produits condensés (G') contenant des monomères de styrène et d'autres composants, pouvant être obtenu à partir de l'étape d) du procédé selon au moins l'une des revendications 1 à 16.

18. Liquide contenant des monomères de styrène, obtenu par le procédé selon au moins l'une des revendications 1 à 16.

19. Liquide contenant des monomères de styrène selon la revendication 18, **caractérisé en ce que** la teneur en composés halogénés est inférieure à 1 000 ppm, de préférence inférieure à 500 ppm, et particulièrement inférieure à 100 ppm, par rapport au poids total du liquide contenant des monomère de styrène.

20. Utilisation d'un composé basique inorganique (B), tel que défini dans au moins l'une des revendications 1 à 16, pour la dégradation de composés organiques halogénés lors de la récupération pyrolytique de monomères à partir de masses polymères, en l'absence d'oxyde d'antimoine (SbO₃), de préférence à partir de masses polymères comprenant des polymères contenant du styrène.
